Europäisches Patentamt

European Patent Office

Office européen des brevets

(11) Veröffentlichungsnummer: **0 423 560 A2**

(12) **EUROPÄISCHE PATENTANMELDUNG**

(21) Anmeldenummer: 90119132.0

(22) Anmeldetag: 05.10.90

(51) Int. Cl.⁵: **A61G 7/05**

(30) Priorität: 19.10.89 DE 8912423 U

(43) Veröffentlichungstag der Anmeldung:
**24.04.91 Patentblatt 91/17**

(84) Benannte Vertragsstaaten:
**AT BE CH DE DK ES FR GB IT LI NL SE**

(71) Anmelder: **FRELU- KRANKENHAUSBEDARF FREDI LUDERICH**

**W-5203 Much-Wellerscheid 11(DE)**

(72) Erfinder: **Gmys, Gisela**
**Messdorfer Strasse 45**
**W-5300 Bonn 1(DE)**

(74) Vertreter: **Schwarz, Klaus-Jürgen, Dipl.-Ing.**
**Gluckstrasse 7**
**W-5300 Bonn 1(DE)**

(54) **Bettschutzdecke.**

(57) Bei bekannten Bettschutzdecken hat es sich gezeigt, daß die Patienten den Gürtel an der Öffnung der Schutzdecke, womit diese den Patienten in Höhe der Taille umgibt, ebenso wie den sich zum kopfseitigen Querrand der Bettschutzdecke erstreckenden verschließbaren Deckenschlitz öffnen und so leicht unter die Decke greifen können. Eine solche Bettschutzdecke sollte daher so ausgebildet sein, daß die Patienten zwar durch die Decke vollständig geschützt sind, ohne jedoch in der Lage zu sein, von selbst unter die Decke zu greifen und sich in unerwünschter Weise zu beschmutzen oder andere Unannehmlichkeiten zu bereiten. Dies wird dadurch erreicht, daß die Bettschutzdecke (2) im Bereich der Öffnung mit einem Hemdenteil (12) für den Patienten einteilig ausgebildet ist. Eine solche Nachthemd-Bettschutzdecke kann wie ein gewöhnliches Nachthemd angelegt und dann wie die herkömmlichen Bettschutzdecken am Bett befestigt werden. Sie fixiert die unruhigen, inkontinenten und verwirrten Patienten im Bett, ohne sie in ihren Bewegungsmöglichkeiten unnötig einzuengen. Die ganze Bettschutzdecke kann in einem Zuschnitt in besonders vorteilhafter Weise aus einem durchgehenden Stück Stoff im Kimono-Schnitt genäht werden und ist dadurch sehr reißfest.

FIG.2

## BETTSCHUTZDECKE

Die Erfindung betrifft eine Bettschutzdecke für inkontinente und unruhige Patienten mit einer Öffnung für den Körper des Patienten und mit Anschlußelementen zum lösbaren Anschluß der Schutzdecke an Teilen des Bettes.

Eine derartige bekannte Bettschutzdecke (DE-GM 83 00 982) weist eine Öffnung für den Körper des Patienten auf, die auf die Körper- bzw. Taillenweite des Patienten einstellbar ist. Am Umfang der Öffnung kann ein in seiner Weite verstellbarer Gürtel befestigt sein, wobei an dem einen losen Gürtelende eine Knopfreihe und an dem anderen losen Gürtelende eine Knopflochreihe zum Verschluß angeordnet sein kann. Die Bettschutzdecke kann außerdem einen verschließbaren Deckenschlitz aufweisen, der sich von der Öffnung bis zum Außenrand der Decke, und zwar vorzugsweise bis zum kopfseitigen Querrand, erstreckt und mittels eines Bendelverschlusses, eines Knopf- oder Reißverschlusses oder dergleichen verschließbar sein kann. Außerdem können an jeder der vier Ecken der Decke sowie an ihren Längsrändern beiderseits der Öffnung für den Patienten Anschlußelemente, beispielsweise in Form von Bendelpaaren angeordnet sein.

Diese bekannte Bettschutzdecke ermöglicht zwar eine weitgehend freie Körperbewegung des Patienten, z.B. auch ein Sitzen desselben sowie einen Wechsel von der Rückenlage in die Seitenlage und umgekehrt. Außerdem besteht auch die Möglichkeit, Schwerkranke Patienten, die sich ohne fremde Hilfe nicht mehr bewegen können, in die Seitenpositionen umzulagern. Bei der Verwendung solcher Bettschutzdecken hat es sich jedoch gezeigt, daß die Patienten den Gürtel an der Öffnung der Schutzdecke und auch den zum kopfseitigen Querrand sich erstreckenden verschließbaren Deckenschlitz öffnen und so unter die Decke greifen können.

Der Erfindung liegt die Aufgabe zugrunde, eine Bettschutzdecke nach dem Oberbegriff des Anspruches 1 dahingehend zu verbessern, daß die Patienten zwar durch die Decke vollständig geschützt sind, ohne jedoch in der Lage zu sein, von selbst unter die Decke zu greifen und sich in unerwünschter Weise zu beschmutzen oder andere Unannehmlichkeiten zu bereiten.

Diese Aufgabe wird erfindungsgemäß dadurch gelöst, daß die Bettschutzdecke im Bereich der Öffnung mit einem Hemdenteil für den Patienten einteilig ausgebildet ist.

Eine solche Nachthemd-Bettschutzdecke wird wie ein gewöhnliches Nachthemd angelegt und dann wie die herkömmlichen Bettschutzdecken am Bett befestigt. Sie fixiert die unruhigen, inkontinenten und verwirrten Patienten im Bett, ohne sie in ihren Bewegungsmöglichkeiten unnötig einzuengen.

Durch ihren großzügigen, weiten Zuschnitt bietet eine solche Nachthemd-Bettschutzdecke ein großes Maß an Bewegungsfreiheit im Sitzen und im Liegen. Sie hindert jedoch verwirrte Patienten daran, über das Bettgitter zu steigen und vermindert so die Verletzungsgefahr. Ebenso verhindert sie, daß verwirrte Patienten sich selbst, das Bett, das Zimmer usw. mit ihren Fäkalien verschmieren, weil der Patient gar keinen Zugang zu diesen hat.

Zum Trockenlegen brauchen nur die drei als Anschlußelemente an den Längsrändern der Bettschutzdecke vorhandenen Haltebänder gelöst zu werden. Der Patient kann dann zur Seite gedreht und die Unterlagen können bequem gewechselt werden.

Besonders bequem für den Patienten ist es, wenn der Hemdenteil einen Halsausschnitt aufweist, dessen Weite etwa dem Kopfumfang des Patienten entspricht, wobei der Halsausschnitt einen V-förmigen Schlitz aufweisen kann, ohne daß die Schutzfunktion der Bettschutzdecke hierdurch beeinträchtigt würde.

Für die Haltbarkeit der Bettschutzdecke ist es weiterhin vorteilhaft, wenn der Halsausschnitt durch einen knopflosen Kragen verstärkt ist, während die Schutzfunktion für den Patienten auch noch dadurch verbessert werden kann, daß der Hemdenteil lange Ärmel mit ringförmig geschlossenen knopflosen Manschetten aufweist.

Für die Haltbarkeit einer solchen Bettschutzdecke ist es ferner vorteilhaft, daß der gesamte Rand der Schutzdecke mit einem Verstärkungsband versehen ist, und die Bequemlichkeit einer solchen Bettschutzdecke für den Patienten kann noch dadurch weiter verbessert werden, daß sie im Bereich des Hemdenteiles breiter als am Kopf- und am Fußende ist, wodurch der Patient eine ausreichende Bewegungsfreiheit hat und sich nicht eingeengt fühlt.

Ferner kann die Haltbarkeit der Bettschutzdecke auch noch dadurch erhöht werden, daß die Längsränder der beiden Ärmelteile bis zu den Längsseiten der Schutzdecke fest miteinander vernäht und mit Verstärkungsband unterlegt sind. Hierbei können mittlere Anschlußelemente an den Verbindungsnähten an der Unterseite der Ärmelteile und an den beiden Längsseiten der Schutzdecke angebracht sein.

Schließlich kann die Bettschutzdecke in besonders vorteilhafter Weise als Zuschnitt aus einem durchgehenden Stück Stoff ausgebildet sein und zwischen ihrem Kopfteil und ihrem Fußteil insgesamt vier einander beiderseits einer gedachten Längsmittellinie spiegelbildlich gegenüberliegende Randausschnitte aufweisen, zwischen denen sich die beiden Ärmelteile des Hemdenteils beiderseits des mittigen Halsausschnittes nach außen erstrekken, was den besonderen Vorteil hat, daß die ganze Bettschutzdecke mit einem solchen Zuschnitt aus einem durchgehenden Stück Stoff im Kimono-Schnitt genäht werden kann und insgesamt sehr reißfest und damit äußerst strapazierfähig ist.

Der Zuschnitt ist dabei vorteilhafterweise so geformt, daß das Kopfteil und das Fußteil sich von ihren Endkanten zum Hemdenteil hin etwa trapezförmig verbreitern.

Zur Anfertigung einer derartigen Nachthemd-Bettschutzdecke wird für den Zuschnitt eine Stoffbahn von etwa 160 cm Breite und etwa 240 bis 260 cm Länge benötigt, wobei die Nähte unter den Ärmeln ebenso wie der gesamte Rand der Bettschutzdecke mit einem etwa 2,5 bis 3 cm breiten Wäscheband oder einem ähnlichen Verstärkungsmaterial unterlegt werden kann.

Die Haltebänder zur Befestigung der Bettschutzdecke am Bett werden jeweils an den vier Ecken und etwa in der Mitte der Längsseiten im Bereich des Hemdenteils angebracht. An dem Halsausschnitt, der gerade so groß ist, daß der Kopf des Patienten hindurch paßt, wird ein Kragen angenäht, der den Halsausschnitt verstärkt, aber andererseits auch dafür sorgt, daß die Bettschutzdecke optisch wie ein Nachthemd wirkt. Die langen Ärmel werden an ihren Enden - ebenfalls ohne Knöpfe leicht gerafft und mit ringförmig geschlossenen Manschetten zusammengefaßt.

Ein bevorzugtes Ausführungsbeispiel der Erfindung ist in der Zeichnung schematisch dargestellt. Es zeigen

Fig. 1 einen Zuschnitt für eine erfindungsgemäße Nachthemd-Bettschutzdecke,

Fig. 2 eine solche fertige Nachthemd-Bettschutzdecke in einer Gebrauchslage in perspektivischer Darstellung und

Fig. 3 die Anordnung einer solchen Nachthemd-Bettschutzdecke für unruhige und inkontinente Patienten an einem Bett mit aufstellbarem Kopfteil.

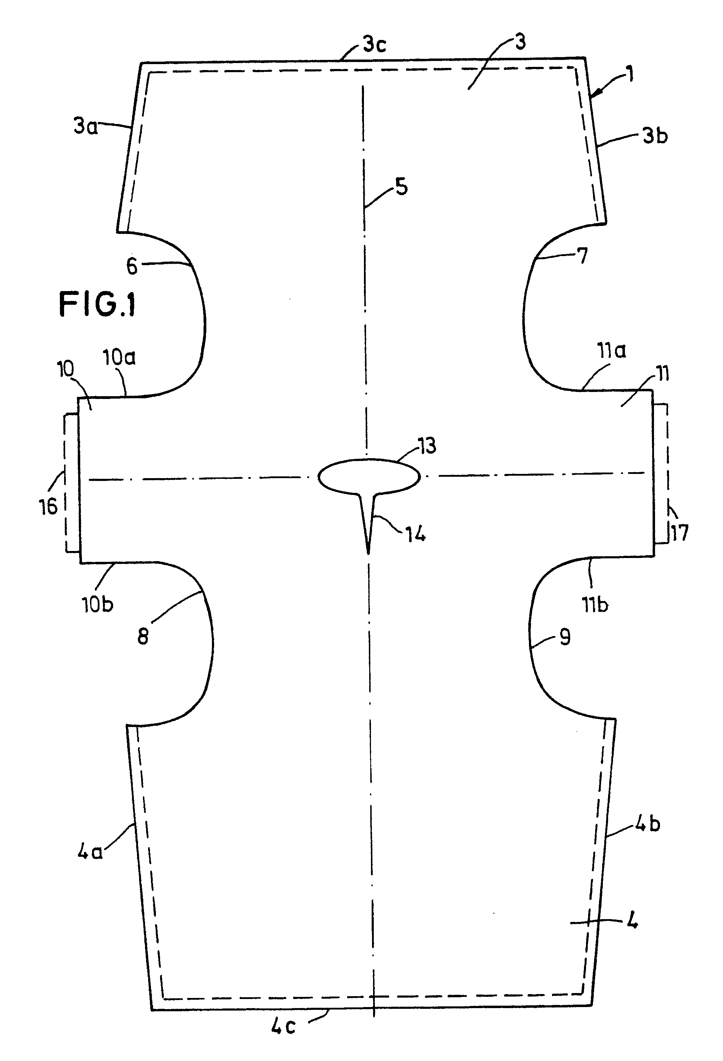

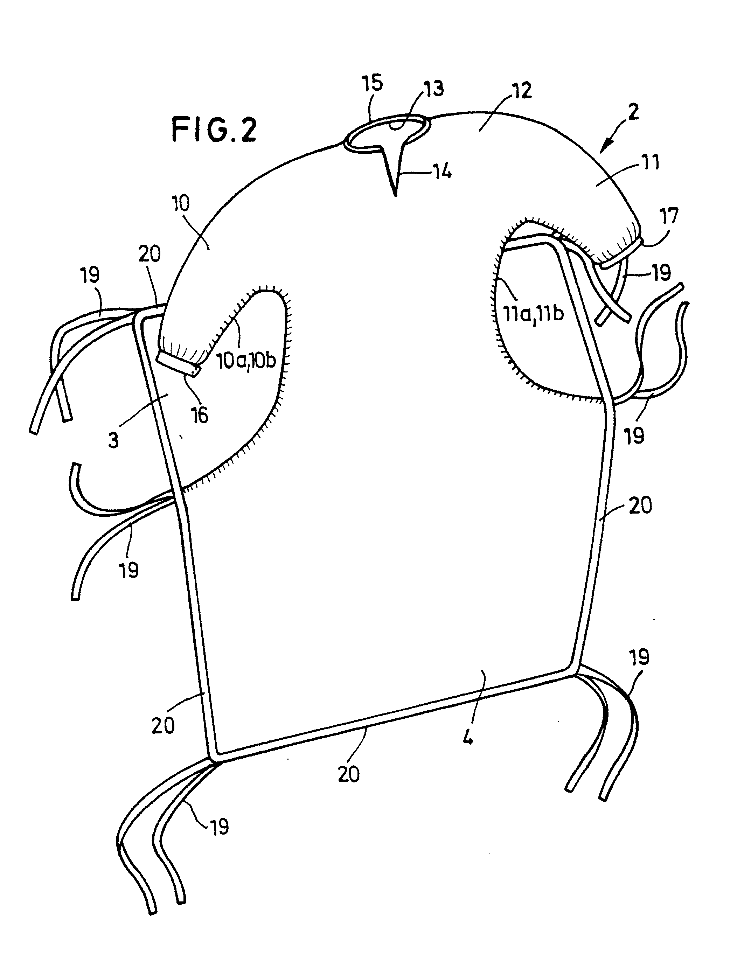

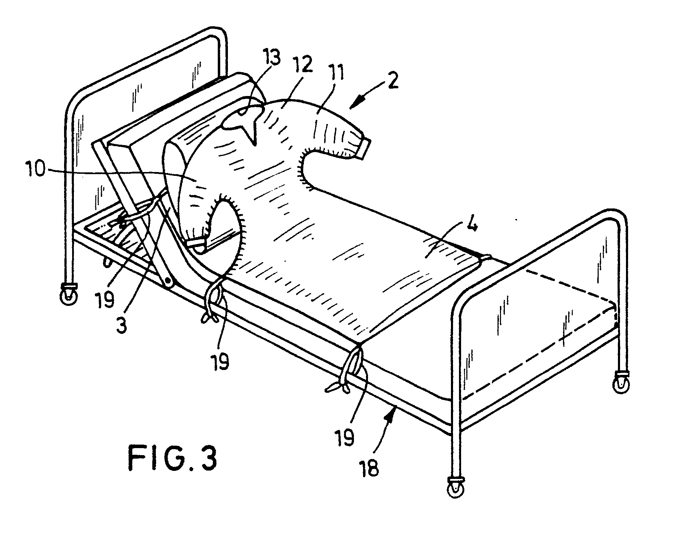

Wie in Fig. 1 gezeigt ist, weist der Zuschnitt 1 für eine Nachthemd-Bettschutzdecke 2 gemäß Fig. 2 und 3 zwischen seinem Kopfteil 3 und seinem Fußteil 4 insgesamt vier einander beiderseits einer gedachten Längsmittellinie 5 spiegelbildlich gegenüberliegende Randausschnitte 6, 7, 8, 9 auf, zwischen denen sich zwei Ärmelteile 10, 11 für einen Hemdenteil 12 (Fig. 2 und 3) beiderseits eines mittigen Halsausschnittes 13 bis über die beiden Längs-Außenränder 3a, 3b bzw. 4a, 4b sowohl des Kopfteils 3 als auch des Fußteils 4 nach außen erstrecken. Das Kopfteil 3 und das Fußteil 4 verbreitern sich von ihren Endkanten 3c bzw. 4c zum Hemdenteil 12 hin etwa trapezförmig, um dem Patienten im Bereich des Hemdenteils 12 ausreichende Bewegungsmöglichkeiten zu bieten.

Wie anhand von Fig. 2 und 3 im einzelnen zu erkennen ist, ist die fertige Nachthemd-Bettschutzdecke mit dem Hemdenteil 12 für den Patienten einteilig ausgebildet, wobei der Hemdenteil 12 einen Halsausschnitt 13 aufweist, dessen Weite etwa dem Kopfumfang des Patienten entspricht. Der Halsausschnitt 13 weist einen V-förmigen Schlitz 14 auf und ist durch einen knopflosen Kragen 15 verstärkt.

Der Hemdenteil 12 hat außerdem lange Ärmel 10,11 mit ringförmig geschlossenen knopflosen Manschetten 16, 17, und für die Befestigung der Bettschutzdecke 2 an Teilen eines Bettes 18 (Fig. 3) sind mindestens sechs Anschlußelemente 19 in Form von Bendelpaaren oder dergleichen vorgesehen, die an den vier Ecken der Bettschutzdecke 2 und jeweils dazwischen an beiden Längsseiten etwa im Bereich des Hemdenteils 12 angeordnet sind.

Der gesamte Rand der Bettschutzdecke ist mit einem Verstärkungsband 20 versehen, und auch im fertigen Zustand ist die Bettschutzdecke 2 im Bereich des Hemdenteils 12 breiter als am Kopf- und am Fußende.

Um die Bettschutzdecke 2 besonders haltbar zu machen, sind die Längsränder 10a, 10b bzw. 11a, 11b der beiden Ärmelteile 10, 11 bis zu den Längsseiten der Schutzdecke 2 fest miteinander vernäht und ebenfalls mit Verstärkungsband unterlegt. Außerdem sind die mittleren Anschlußelemente 19 am Hemdenteil 12 im Bereich der Verbindungsnähte an der Unterseite der Ärmelteile 10, 11 an den beiden Längsseiten der Bettschutzdecke angebracht.

Liste der Bezugszeichen

1 Zuschnitt
2 Nachthemd-Bettschutzdecke
3 Kopfteil
3a Längs-Außenrand
3b Längs-Außenrand
3c Endkante
4 Fußteil
4a Längs-Außenrand
4b Längs-Außenrand
4c Endkante
5 Längsmittellinie
6 Randausschnitt
7 Randausschnitt
8 Randausschnitt
9 Randausschnitt
10 Ärmelteil

10a Längsrand
10b Längsrand
11 Ärmelteil
11a Längsrand
11b Längsrand
12 Hemdenteil
13 Halsausschnitt
14 V-förmiger Schlitz
15 Kragen
16 Manschette
17 Manschette
18 Bett
19 Anschlußelement
20 Verstärkungsband

**Ansprüche**

1. Bettschutzdecke für inkontinente und unruhige Patienten mit einer Öffnung für den Körper des Patienten und mit Anschlußelementen zum lösbaren Anschluß der Schutzdecke an Teilen des Bettes, **dadurch gekennzeichnet,** daß die Bettschutzdecke (2) im Bereich der Öffnung mit einem Hemdenteil (12) für den Patienten einteilig ausgebildet ist.

2. Bettschutzdecke nach Anspruch 1, **dadurch gekennzeichnet,** daß der Hemdenteil (12) einen Halsausschnitt (13) aufweist, dessen Weite etwa dem Kopfumfang des Patienten entspricht.

3. Bettschutzdecke nach Anspruch 1 und 2, **dadurch gekennzeichnet,** daß der Halsausschnitt (13) einen V-förmigen Schlitz (14) aufweist.

4. Bettschutzdecke nach Anspruch 1 und 2, **dadurch gekennzeichnet,** daß der Halsausschnitt (13) durch einen knopflosen Kragen verstärkt ist.

5. Bettschutzdecke nach Anspruch 1, **dadurch gekennzeichnet,** daß der Hemdenteil (12) lange Ärmel (10, 11) mit ringförmig geschlossenen knopflosen Manschetten (16, 17) aufweist.

6. Bettschutzdecke nach Anspruch 1, **dadurch gekennzeichnet,** daß der gesamte Rand der Schutzdecke (2) mit einem Verstärkungsband (20) versehen ist.

7. Bettschutzdecke nach einem oder mehreren der Ansprüche 1 bis 6, **dadurch gekennzeichnet,** daß sie im Bereich des Hemdenteiles (12) breiter als am Kopf- und am Fußende (3, 4) ist.

8. Bettschutzdecke nach Anspruch 1 und 5, **dadurch gekennzeichnet,** daß die Längsränder (10a, 10b bzw. 11a, 11b) der beiden Ärmelteile (10, 11) bis zu den Längsseiten der Schutzdecke (2) fest miteinander vernäht und mit Verstärkungsband unterlegt sind.

9. Bettschutzdecke nach Anspruch 8, **dadurch gekennzeichnet,** daß mittlere Anschlußelemente (19) an den Verbindungsnähten (10a, 10b bzw. 11a, 11b) an der Unterseite der Ärmelteile (10, 11) an den beiden Längsseiten der Schutzdecke (2) angebracht sind.

10. Bettschutzdecke nach einem der vorhergehenden Ansprüche, **dadurch gekennzeichnet,** daß sie als Zuschnitt aus einem durchgehenden Stück Stoff ausgebildet ist, und daß sie zwischen ihrem Kopfteil (3) und ihrem Fußteil (4) insgesamt vier einander beiderseits einer gedachten Längsmittellinie (5) spiegelbildlich gegenüberliegende Randausschnitte (6, 7, 8, 9) aufweist, zwischen denen sich die beiden Ärmelteile (10, 11) des Hemdenteils (12) beiderseits des mittigen Halsausschnittes (13) nach außen erstrecken.

11. Bettschutzdecke nach Anspruch 10, **dadurch gekennzeichnet,** daß das Kopfteil (3) und das Fußteil (4) sich von ihren Endkanten (3c, 4c) zum Hemdenteil hin etwa trapezförmig verbreitern.

FIG.1

FIG.2

FIG.3